Europäisches Patentamt

**European Patent Office**

**Office européen des brevets**

(11) Publication number: **0 166 124**
A2

# EUROPEAN PATENT APPLICATION

(21) Application number: **85105374.4**

(22) Date of filing: **30.04.85**

(51) Int. Cl.⁴: **A 61 B 19/08,** A 61 F 9/00

(30) Priority: **02.05.84 US 606116**

(71) Applicant: **SURGIKOS INC., 501 George Street, New Brunswick New Jersey 08903 (US)**

(43) Date of publication of application: **02.01.86 Bulletin 86/1**

(72) Inventor: **Scrivens, George W., 2007 Royal Club Court, Arlington Tx. 76017 (US)**
Inventor: **Waller, Charles M., 804 Live Oak Lane, Arlington TX 76016 (US)**

(84) Designated Contracting States: **AT BE CH DE FR GB IT LI NL SE**

(74) Representative: **Groening, Hans Wilhelm, Dipl.-Ing., Siebertstrasse 4 Postfach 860 340, D-8000 München 86 (DE)**

(54) **Ophthalmology drape.**

(57) A surgical drape for ophthalmology procedures is disclosed. The upper portion (15) of the drape is constructed of a plastic film. There is an absorbent reinforcing area (20) in the drape and a fenestration (21) through the reinforcing area (20). There is a border of plastic film between the fenestration (21) and the absorbent material in the reinforcing area (20).

EP 0 166 124 A2

OPHTHALMOLOGY DRAPE

Background of the Invention

Field of the Invention

The present invention generally relates to a surgical drape and particularly to a surgical drape to be used in ophthalmology procedures. The drape includes a main body portion which can be made of a non-woven fabric and a head covering portion which is made of a plastic material which is non-linting.

Description of the Prior Art

Previous procedures employed to drape ophthalmological patients included the use of multiple component surgical drapes. Some of these drapes had small incise films in a portion of the drape. An incise film is a clear plastic film with adhesive on the body contact side of the film. The incise film would be positioned over the eye of the patient and the surgical procedure would be performed through the incise film. The incise film would be cut away to expose the portion of the eye area of the patient's head on which the surgical procedure would be performed. In order to completely drape the patient, it was often necessary to use draping towels and other surgical drapes to completely cover the patient. The problem with many of these drapes is that they would cause linting, which is particularly undesirable in ophthalmological procedures. Many of these procedures are done with operating room microscopes, and lint can interfere with the surgeons view through the microscope. The presence of lint also causes a problem with post-operative infection and other problems caused by small fibrous particles of lint in the operative site.

## Summary of the Invention

The object of the present invention is to provide a surgical drape which is specifically adapted for use in ophthalmological procedures. The drape avoids the linting problem of prior art drapes and provides a relatively simple draping technique to allow the surgical staff to quickly drape the patient in an aseptic manner.

## Brief Description of the Drawings

Fig. 1 is a top plan view of the surgical drape of the present invention.

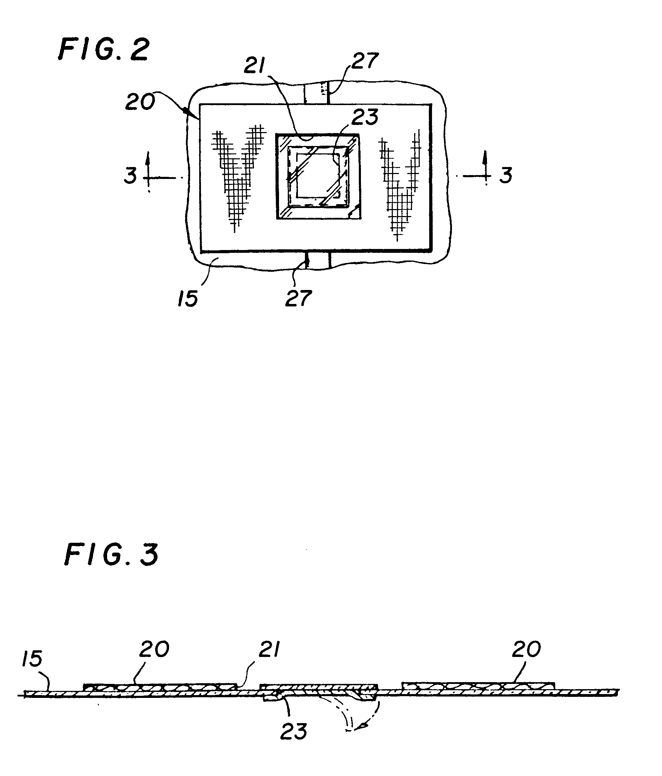

Fig. 2 is a fragmentary plan view of the central region in the upper portion of the drape of the present invention.

Fig. 3 is a cross-sectional view taken along lines 3-3 of Fig. 2.

Fig. 4 is an exploded view of the reinforcement area of the drape.

Fig. 5 is a top plan view of a modification of the drape of the present invention.

## Detailed Description of the Present Invention

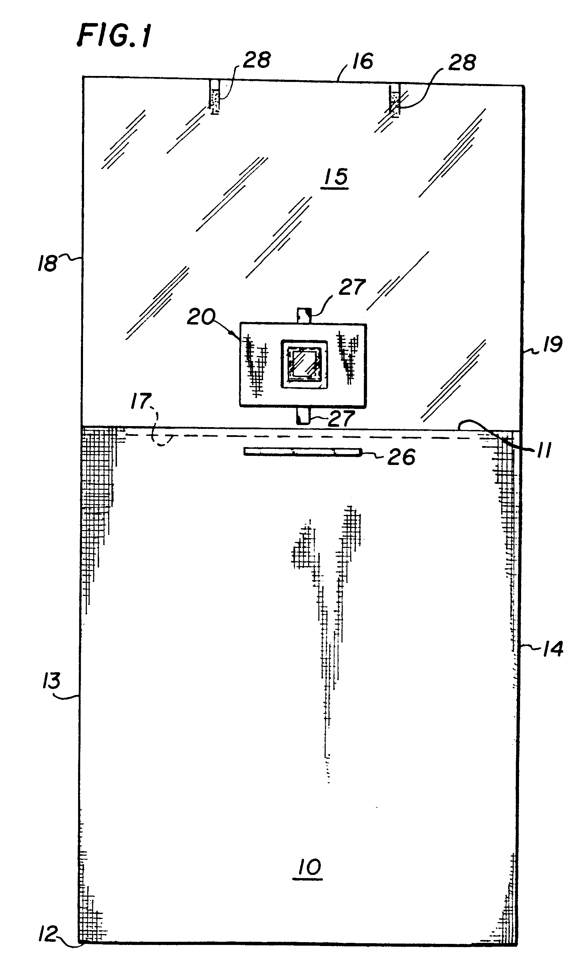

As shown in Figure 1 the ophthalmological drape of the present invention comprises a surgical drape having a main body portion 10 which has a top edge 11, bottom edge 12 and two opposing side edges 13 and 14. The main body portion of the drape is a sufficient size to cover the torso of the patient from the neck to the feet of the patient. The main body of the drape can be made with a non-woven fabric. The non-woven fabric is usually treated

SU-51

with a water-repellant finish to provide repellency to prevent strike through of operating room liquids through the drape to the patient.

There is a head portion 15 of the drape which has a top edge 16, bottom edge 17 and two opposing side edges 18 and 19. The bottom edge of the head portion 15 of the drape is attached or secured to the top edge of the main body of the drape by adhesive or by heat sealing or other means. The top portion of the drape is made of a plastic film which is preferably transparent. This plastic film will be referred to as the first plastic film. There is a reinforcement area 20 located centrally between the side edges of the drape and towards the bottom edge of the head portion of the drape. This reinforcement area is a layer of a non-woven fabric which has absorbent properties to absorb any liquids that may be present during the surgical procedure and to prevent such liquids from running into the operative site. The absorbent material is non-linting and will shed very little, if any, fiber if contacted by the surgical staff. In the center of the reinforcement area, there is a fenestration or opening 21 through the non-woven fabric and a smaller fenestration 23 through the first plastic film of the head portion of the drape. The fenestration is approximately 2,54 cm by 2,54 cm in the reinforcement area of the drape. The fenestration in the reinforcement area exposes a portion of the underlying film of the head portion of the drape. Located over the small fenestration there is an incise film which overlaps a portion of the plastic sheet. The incise film will be referred to as the second plastic film. The incise film is a clear plastic film with adhesive on the body contact side of the film. The incise film may be apertured or it may be left for the surgeon to fenestrate. The particular dimensions of the fenestrations are not critical, and different size fenestrations may be used. A release sheet

51

is placed over the adhesive, and it is removed prior to the application of the drape to the patient. This construction results in a surgical drape in which the reinforcement area does not extend to the edges of the fenestration. Although the particular physical dimensions of the drape are not critical, a drape having the following dimensions has been found to be acceptable. The main body portion of the drape, from the top edge 11 to the bottom edge 12, is approximately 203 cm long. The head portion of the drape, from its bottom edge 17 to the top edge 16, is approximately 101 cm. The width of the drape, both the main body portion and the head portion, is approximately 152 cm. The reinforcement area is approximately 25 cm by 40 cm. The fenestration in the reinforcement area is 15,2 cm square, and the fenestration in the plastic sheet is 7,6 cm square. There is a 2,5 to 7,6 cm border of plastic between the reinforcement fabric and the incise film over the fenestration in the film portion of the drape. This border may be more than 7,6 cm but should not be less than 1,3 cm. The space between the operative fenestration and the reinforcement fabric provides security to prevent lint from the reinforcement area fabric from being introduced into the operative site.

Near the upper edge of the body portion 10 of the drape, there is a metal strip 26 which is generally of the same width as the reinforcement area. This metal strip is malleable and can be readily bent into an arcuate shape to space the drape away from the mouth and nose of the patient to enable the patient to breathe normally during the surgical procedure.

There may be tabs 27 secured to the edges of the reinforcement area to provide a position to secure suction devices or cautering devices to the surgical drape without the fear of penetration of the drape by the clamps that are used to secure such devices to the drape. In addition at the top edge of the drape, that is the top edge of the head portion of the drape, there may be adhesive tabs 28 to secure the drape to rods to form an anesthesia screen.

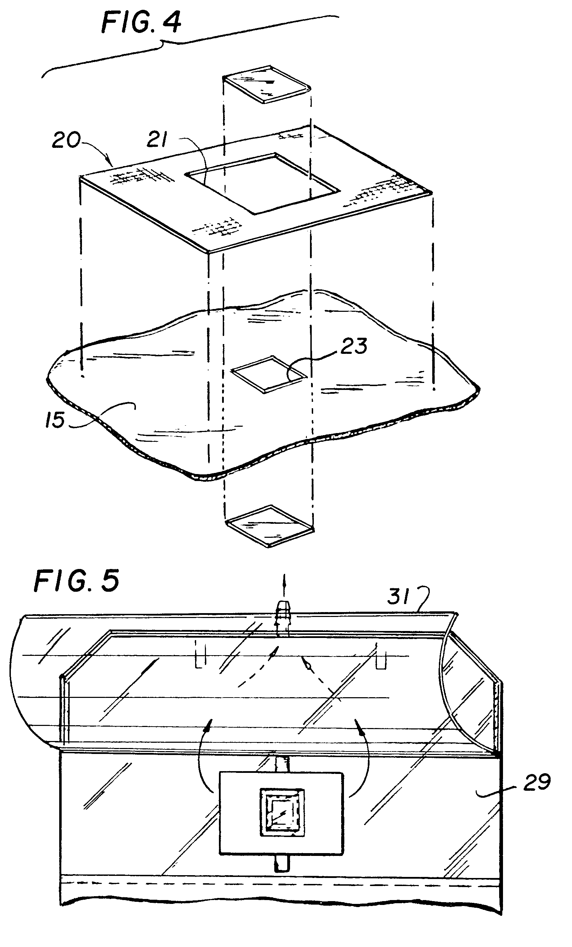

Fig. 5 shows a modification of the drape. In this modification an additional piece or layer of plastic film 29 is secured to the upper surface of the head portion of the drape along top edge 16 and side edges 18 and 19 to form a drainage bag. The bag which is used to collect irrigation and other liquids may be present during the surgical procedure. There may be a flexible flap 31 which is not secured or attached to the film 29. This flap is secured to a stand or screen support to insure that the bag can be held in an open position to collect liquids. There is a drain plug 30 at the top edge of the drape which can be connected to drainage tubing to remove the fluids from the drape. The plug 30 may be located in the lower surface of the bag.

## Claims

1. A surgical drape for use in ophthalmological procedures comprising a body portion comprising a generally rectangular, flexible sheet having a top edge, bottom edge and two opposing side edges, a head covering portion comprising a generally rectangular sheet having a top edge a bottom edge and two opposing side edges, the bottom edge of the head covering portion being attached to the top edge of the body portion, the head covering portion comprising a first plastic film having a reinforcing area located between the side edges, said reinforcing area comprising a layer of non-linting absorbent material, a fenestration through the central region of the reinforcing layer, a fenestration in the plastic film which is smaller than the fenestration in the reinforcing layer forming a border of plastic film between the fenestration in the reinforcing layer and the fenestration in the first plastic film, a transparent second plastic film having adhesive on the lower surface overlying the fenestration in the first plastic film.

2. The surgical drape of Claim 1 in which the first plastic film is transparent.

3. The surgical drape of Claim 1 in which there is a strip of malleable metal attached to the drape near the upper edge of the main body portion of the drape.

4. The surgical drape of Claim 1 in which the border between the fenestration in the reinforcing layer and the fenestration in the first plastic film is between 1,3 cm and 7,6 cm.

5.  The surgical drape of Claim 1 in which there is an additional layer of film secured to the top and side edges of the head portion of the drape to form a fluid collection bag.

6.  The drape of Claim 5 in which the layer of film has a flexible flap which is free of attachment to the first plastic film.

U-51

*FIG.1*

FIG. 2

FIG. 3

FIG. 4

FIG. 5